# EUROPÄISCHE PATENTANMELDUNG

(11) **EP 1 927 338 A1**
(43) Veröffentlichungstag der Anmeldung: **04.06.2008**
(21) Anmeldenummer: 07021673.4
(22) Anmeldetag: 08.11.2007
(51) Int. Cl.: A61K 8/19, A61K 8/24, A61K 8/65, A61Q 11/00

(54) **Mittel zum Schutz von Zahnflächen im Anschluss an konventionelle Bleichverfahren durch biomimetische Abscheidung von Fluorapatit**

(30) Priorität: 21.11.2006 DE 102006055223
(71) Anmelder: Heraeus Kulzer GmbH, 63450 Hanau (DE)
(72) Erfinder: Busch, Susanne Dr., 61267 Neu Anspach (DE); Hoffmann, Marcus Dr., 61250 Usingen (DE); Utterodt, Andreas Dr., 61267 Neu Anspach (DE); Mätzig, Christoph Dr., 63526 Erlensee (DE)
(74) Vertreter: Kühn, Hans-Christian

(57) **Zusammenfassung**

Die Erfindung betrifft ein Mittel zum Schutz von Zahnflächen im Anschluss an konventionelle Bleichverfahren durch biomimetische Abscheidung von Fluorapatit, aufweisend
**A** mindestens einen vorkonfektionierter 50-1000 µm dicken Gelfilm A enthaltend
A1 mindestens einen Gelbildner,
A2 Wasser oder ein Gemisch aus Wasser und einem organischen Lösungsmittel,
A3 Phosphat- oder Hydrogenphosphationen,
A4 optional Fluorid,
A5 optional mindestens eine Aminosäure,
A6 ggf. eine Carbonsäure oder ein Puffersystem für einen der pH-Werte von 4 bis 7.

**B** mindestens einen 50 µm bis 5 mm dicken Gelfilm B enthaltend
B1 mindestens einen Gelbildner,
B2 Wasser,
B3 Calciumionen Cₐ²⁺.

## Beschreibung

Die Erfindung betrifft Mittel zum Schutz von Zahnflächen im Anschluss an konventionelle Bleichverfahren durch biomimetische Abscheidung von Fluorapatit.

### Hintergrund

Zur Aufhellung von Zähnen werden vorwiegend Bleichsysteme eingesetzt die mittels starken Oxidationsmitteln wirken. Die Konzentrationen liegen je nach Applikationsform zwischen 10-35 % Peroxid. Insbesondere werden konz. Wasserstoffperoxidlösungen oder Carbamidperoxid eingesetzt. Die Wirkungsweise beruht auf der oxidativen Entfärbung von eingelagerten Farbstoffen. Sie haben eine aggressive Wirkung auf die Mundschleimhaut, so dass der Kontakt unbedingt vermieden werden muss. Starke Oxidationsmittel könnten jedoch auch strukturrelevante Proteine im Schmelz degradieren. Der Gehalt an natürlichen Makromolekülen im Zahnschmelz ist abhängig vom Reifungsgrad und beträgt beim Erwachsenen ca. 1-2 Gew.%. Proteine finden sich bevorzugt auf der Oberfläche der schmelzprismatischen Einheiten, können aber auch in der Kristallstruktur integriert sein. Kennzeichen der Biomineralisation sind Kompositsysteme aus anorganischem Mineral und organischen Komponenten (bevorzugt Proteine und Polysaccharide). Das spezifische Zusammenspiel führt zu erhöhter Bruchfestigkeit. Wird die organische Komponente chemisch entfernt, ist mit einer Versprödung zu rechnen. Bei der oxidativen Zahnaufhellung ist zudem die Zugänglichkeit für Verunreinigungen zumindest in den ersten Tagen nach der Behandlung erhöht und die Schmerzempfindlichkeit teilweise stark erhöht. Vermutlich wird durch die Oxidation der biologischen Matrix ein feines Porensystem durchgängig, das zuvor von Protein ausgefüllt war. Dadurch können Schmerzreize leichter an den Zahnnerv geleitet werden und unerwünschte Fremdstoffe besser eindiffundieren. Das würde erklären, warum Bleichanwendungen häufig wiederholt werden müssen und behandelte Zähne für Entzündungen empfänglich sein können. Manche Bleaching-Systeme arbeiten bei niedrigen pH-Werten, was darüber hinaus zu einer Demineralisation führen kann und eine zusätzliche Schwächung für den Zahn bedeutet. Topographische Untersuchungen an gebleichten Humanzähnen zeigen eine wahrnehmbare Zunahme der natürlichen Porosität (cf Bitter N. C. and Sanders J., L.: The effect of four bleaching agents on the enamel surface: A scanning electron microscopic study. Quintessence Int 1993, 24: 817-24). Daraus resultiert eine erhöhte Empfindlichkeit gegenüber Säureangriffen. Obwohl die Härte des Zahns nach der Bleichprozedur keine messbare Verminderung zeigt, belegen Studien an gebleachten Zähnen eine deutliche Verminderung der Mikrohärte und verstärkte Auflösungserscheinungen nach simulierten Demineralisations-Remineralisationszyklen. Der Effekt wird zwar durch die Gabe von Fluorid etwas vermindert, aber die Schwächung nicht aufgehoben (cf Attin T., Kocabiyik M., Buchalla W., Hanning C., Becker K.: Susceptibility of Enamel Surfaces to Demineralisation after Application of Fluoridated Carbamide Peroxide Gels, Caries Res 2003: 37: 93-99). Eine andere Studie belegt, dass die Zug- und Bruchfestigkeit von Schmelz nach Einwirkung von Bleichlösungen um bis zu 30% abnimmt (see: Cavalli, V. et al: Effect of carbamide peroxide bleaching agents on tensile strength of human enamel. Dental Materials (2004) 20, 733-9). Eine Schmerzstudie belegt, dass nach einer Behandlung mit 35% Wasserstoffperoxidlösung 2/3 der Patienten über mäßige bis starke Schmerzen berichteten, die bis zu 48 Stunden nach der Behandlung andauerten (see Nathanson D, Parra C.: Bleaching vital teeth: a review and clinical study. Compend. Contin. Educ. Dent. 1987; 8(7): 490-7). Die Studien ergaben zwar keine eindeutigen Hinweise auf eine irreversible Pulpa-Schädigung, im Tierversuch traten aber nach dem Bleichen von vitalen Hundezähnen Fälle von massiven Entzündungen, Dentinresorption bis hin zum Absterben der Pulpa auf (see Nathanson, D.: Vital Tooth bleaching: Sensitivity and Pulpal considerations, JADA, 128 (April 1997) 41-4.) Vorraussetzung für die konventionelle Bleaching Prozedur sind in jedem Fall gesunde Zähne. Liegen hypersensible Zähne, freiliegende Zahnhälse oder Karies vor, kann das Bleaching den Zahnnerv irreversibel schädigen. Denkbar ist ferner, dass sich auch bei gesunden Zähnen Schäden durch wiederholtes Bleachen akkumulieren.
Um diesem Problem entgegen zu wirken wird versucht, freigelegte Mikroporen durch biomimetische Remineralisierung zu verfüllen und darüber hinaus eine apatitische Schutzschicht aufzubringen. Da Apatit und insbesonders Fluorapatit bereits ab pH 5 das thermodynamisch stabilste Calciumphosphat darstellt, lässt sich leicht zeigen, dass bei physiologischem pH-Wert in Gegenwart von Calcium- und Phosphat-Ionen bevorzugt Apatit gebildet wird. Um der Kompositnatur des biologischen Vorbildes zu entsprechen, findet die Mineralisation in Gegenwart eines organischen Gels statt. Das hat zur Folge, dass Makromoleküle in das Apatitgefüge integriert werden.

### Stand der Technik

In der Patentliteratur sind einige Verfahren beschrieben, die zum Ziel haben, die zahnsubstratschwächende Wirkung des Bleachings durch Remineralisation auszugleichen. Dem Oxidationsmittel werden entweder calcium- und phosphathaltige Salze zugefügt werden, oder das Bleichmittel selbst stellt ein calcium- und/oder phosphathaltiges Peroxid dar. US 6,521,251 beschreibt eine Zusammensetzung, die neben Carbamid Calciumphosphate enthält, deren Löslichkeit etwas besser ist, als die von Apatit, wie Mono-Di- oder Tricalciumphosphat. Dennoch sind all diese Calciumphosphate nur wenig wasserlöslich, daher ist eher eine abrasive als eine remineralisierende Wirkung zu erwarten. Tatsächlich beschreibt US 5,851,514 unter anderem die Zugabe von Dicalciumphosphat als Abrasivum. US 6,419,905 erwähnt die Zugabe von Kaliumsalzen (z. B. Phosphate) und Fluorid zum Peroxid. Fluorid ist geeignet aus dem Speichel Calcium- und Phosphat-lonen zu binden, wodurch Fluorapatit ausfällt. Wenn keine weiteren Ionen zugefügt werden, wird auch die Bildung von CaF₂ beobachtet. Die Calciumfluoridparikel können in der Plaque gespeichert werden und über längere Zeit Fluorid abgeben, da sie etwas löslicher sind als Apatit. Jedoch werden vor einem professionellen Bleaching alle Zähne durch intensive Reinigung von der Plaque befreit. JP 20000051804 beschreibt den parallelen Einsatz von konzentrierter Phosphorsäure, konz. H₂O₂ und Fluorapatitpulver. Problematisch erscheint hier der Einsatz der konzentrierten Phosphorsäure, die gesunden Zahnschmelz merklich auflösen kann. Zudem ist die Bleichlösung stark ätzend und darf nicht in Kontakt mit dem Zahnfleisch kommen, was aber in abgeschwächter Form für alle oxidativ wirkenden Zahnaufheller gilt. Zudem führt die wiederholte Anwendung nicht zu einer Zunahme der Remineralisationsschicht. Es wird vermutet, dass sich hier allenfalls aufgelöster Zahnschmelz wieder niederschlägt. Eine säurefreie Anwendung wird in US 20050281759 beschrieben. Als wesentlicher Inhaltsstoff wird hier Calciumperoxophosphat vorgeschlagen. Diese Idee hat den Vorteil, dass eine einzige Substanz aufhellend und remineralisierend wirken soll, da parallel zur Oxidation die Freisetzung von Calcium- und Phosphat-lonen ausgelöst wird. Nicht klar ist, ob die Salze in der verhältnismäßig kurz währenden Einwirkungsdauer einen nennenswerten Zahnaufbau leisten können. US 6,303,104 beschreibt ein oxidationsmittelfreies Zwei-Komponentensystem aus löslichen Calcium- und Phosphatsalzen, das auch eine aufhellende Wirkung haben soll. Die Aufhellung soll durch die Zugabe von Natriumgluconat hervorgerufen werden, welches färbende Metallionen (z. B. Eisen) aus dem Zahnschmelz komplexiert. Beim Vermischen der Komponenten ist sofort mit einer Ausfällung der schwerlöslicher Calciumphosphate zu rechnen und es ist nicht zu erkennen, warum es zu ausgeprägter Remineralisation kommen soll, zumal es sich bei dem Produkt um eine Zahnpasta handelt, die nur wenige Minuten in Kontakt mit den Zahnflächen verbleibt. Der entfärbende Effekt reduziert sich zudem auf komplexierbare Metallionen und wird vermutlich nur in den äußersten Schmelzschichten eine Wirkung zeigen. US 6,102,050 beschreibt eine Zahnseide mit Titandioxidpartikeln, der eine aufhellende, remineralisierende und desensibilisierende Wirkung auf die Interproximalflächen zugeschrieben wird. Hier sollen Titandioxidmikropartikel der Größe 0.1-1.5 µm sowohl als mildes Abrasivum wirken, als auch vom Schmelz absorbiert werden, was mit einer aufhellenden Wirkung verbunden sei. Vermutlich können sich die Partikel allenfalls mechanisch in geeignete Hohlräume einlagern, was keine stabile Verankerung verspricht.

Alle bisher beschriebenen Patente berücksichtigen nicht, dass Biominerale ihre hohe strukturelle Organisation und Stabilität nur erreichen, weil sie sich in Gegenwart von speziellen Biomolekülen bilden, die Mikro- und Makrostrukturierung vorgeben. WO 2005/027863 beschreibt ein Zahnpflegemittel, das über reinigende, remineralisierende, desensibilisierende und aufhellende Wirkung verfügen soll. Als aktive Komponente zur Remineralisation und Aufhellung ist ein nanoskaliger Apatit angegeben, der in Gegenwart einer wässrigen Gelatinelösung gefällt wird und daher Polypeptide eingelagert hat. Diesem Material wird zugesprochen, dass es durch sogenannte "Neomineralisation" auf der Zahnoberfläche einen Schutzfim von dentinähnlicher Struktur bildet, der eine Oberflächenglättung bewirkt und offene Dentintubuli verschließen kann. Erstaunlich ist diese Wirkung, da bevorzugt nur 0.01-2 Gew.% "Nanite" (WO 01/01930) in Zahnpflegemittel enthalten sind. Die Einwirkung der aktiven Substanzen liegt bei nur einigen Minuten täglich. Daher ist nicht mit einer ausgeprägten Mineralabscheidung zu rechnen. Zudem sind nanoskalige Teilchen farblos, so dass die Einlagerung von Nanite nicht zu einer Farbveränderung führen sollte. Ein kontinuierlicher Zuwachs der Filmdicke bei längerer Anwendung des Pflegemittels wird nicht beobachtet. Zudem ist Dentin nicht geeignet, den Zahn vor korrosiven Angriffen zu schützen.

Die Möglichkeit einer kontinuierlich wachsenden FAP-Schicht mit schmelz- oder dentinähnlicher Struktur bietet die in US 20005220724 und DE 1020040545847 beschriebene Technik. Wasserlösliche Phospat- und Fluorid-Salze werden in dem gepufferten Gel A, Calcium-Ionen im Gel B eingearbeitet. Optional durch eine ionenfreie Schutzschicht getrennt, werden die bei physiologischer Temperatur festen Gelatine-Glycerin-Gele unter Erwärmung nacheinander auf Zahnoberflächen aufgetragen. In Abhängigkeit von den Austauschzyklen der Gele kann eine Zunahme der Schichtstärke beobachtet werden. Die Wachstumsgeschwindigkeiten betragen maximal 3-5 µm/Tag. Die biologischen Strukturen des Zahnmaterials werden individuell vom Fluorapatit abgebildet, Hohlräume durch offenliegende Dentintubuli werden jedoch nach einigen Austauschzyklen verschlossen. Hinsichtlich einer Anwendung am Menschen erweist es sich als ungünstig, dass die Gele vor der Applikation erwärmt werden müssen. Durch das Aufbringen der zweiten und dritten Gelschicht können darunter liegende bereits applizierte Gelschichten wieder verflüssigt werden und in unerwünschter Weise mit darüber liegenden Schichten verwirbeln. Insbesondere kleinere Darreichungsformen trocknen bei Exposition an der Luft schnell aus und ein Verflüssigen durch Erwärmung ist dann nicht mehr ohne weiteres möglich. Die Methode erlaubt nicht genau definierte Gelmengen auf den Zahn aufzubringen. Ferner tragen die drei bis zu 6 mm dicken Gelschichten stark auf, was bei Schutzsystemen wie Schienen oder Pflastern zu Problemen führt, da hier Platz für große Gelreservoirs geschaffen werden muss.

### Aufgabenstellung

Ziel ist es, einen Zahnschutz oder eine Regeneration kleinerer Zahndefekte zu erzielen, insbesondere bei der Nachbehandlung oxidativ aufgehellter Zähne.

### Darstellung der Erfindung

Es werden Methoden und Mittel beschrieben, die zur gezielten biomimetischen Mineralisation von Apatit auf Zahnflächen, als nachgelagerte Prozedur der oxidativen Zahnaufhellung, mit dem Ziel des Zahnschutzes, eingesetzt werden können. Es wird erwartet, dass diese reparative Nachbehandlung den aus dem Bleichprozess resultierenden Verlust an Mineralien und Proteinen sowie die erhöhte Sprödigkeit durch das direkte Aufwachsen eines Komposits aus einem bio-organischen Gel kompensiert und eine Verlängerung der Bleichwirkung mit sich führt. Infolge der langen Einwirkdauer der aktiven Mineralien können sich effektiv mit dem Substrat verwachsene, hochgeordnete kristalline Schichten bilden. Die durch den oxidativen Eingriff gebildeten Poren und Risse werden durch die Remineralisation verschlossen, wodurch eine erneute Verfärbung reduziert wird. Wenn die Schutzschicht mit der Zeit abradiert wird, ist eine Akkumulation von neuen Verfärbungen in den äußeren Zahnschmelzschichten ausgeschlossen. Mit dem erfindungsgemäßen Mittel verbunden ist eine Schutzbehandlung von aufgehellten Zähnen mit dem Ziel, unerwünschte Nebenwirkungen des Bleichens zu vermindern bzw. aufzuheben und die Stabilität der Zähne zu verbessern. Die Verfärbungsneigung aufgehellter Zähne kann vermindert werden. Es kann erwartet werden, dass der Zahn nicht nur schneller seine ursprüngliche Stabilität wieder erlangt, sondern dass die zunächst erhöhte Empfindlichkeit deutlich schneller abklingt und der aufhellende Effekt durch die Schutzschicht länger anhält. Durch die mit der geringen Schichtdicke verbundenen verkürzten Diffusionswege der Ionen vermindert sich die Rückvermischung von Phosphat mit Calcium-Salzen, und eine besondere Begünstigung der direkten Zahnoberfläche als heterogene Nukleationsfläche bleibt auch ohne das ionenfreie Schutz-Gel in ausgeprägter Weise erhalten. Durch die definierte Schichtdicke lassen sich reproduzierbar gleiche Gelmengen auf den Zahn auftragen. Die Schicht lässt sich mit SiC-Schleifpapier polieren.

### Detaillierte Beschreibung der Erfindung

Die Erfindung betrifft Mittel zum Schutz von Zahnflächen nach konventionellen Bleichverfahren durch biomimetische Abscheidung von Fluorapatit, aufweisend
**A** mindestens einen vorkonfektionierter 50-1000 µm dicken Gelfilm A enthaltend einen Gelbildner
   Wasser oder ein Gemisch aus Wasser und einem organischen Lösungsmittel
   0.01 -2 mol pro Liter Gel Phosphat oder Hydrogenphosphat,
   ggf. Fluorid,
   ggf. mindestens eine Aminosäure,
   ggf. eine Carbonsäure oder ein Puffersystem für einen der pH-Werte von 4 bis 7,
**B** mindestens eine 50 µm bis 5 mm dicken Gelfilm B enthaltend
   einen Gelbildner,
   Wasser,
   0.05 bis 3 mol/I Calciumionen Ca²⁺.

Die Verwendung solcher dünner, vorkonfektionierter Gelfilme kompensiert die aus US 20005220724 beschriebenen Nachteile, die sich aus dem Einsatz warm aufgegossener Gele ergeben. Die Dicke der Gele A beträgt bevorzugt 150-500 µm. Die Konzentration der Phosphatsalze beträgt bevorzugt 0.08 bis 0.3 mol. Die Dicke der Gele B beträgt bevorzugt 200 bis 600 µm. Die Konzentration der Gele B beträgt bevorzugt 0.13-1 mol/l.

Als Phosphat- oder Hydrogenphosphatquelle kommen vorzugsweise Phosphorsäure oder deren Alkalisalze in Frage.

Überraschenderweise wurde festgestellt, dass sich durch die Verwendung der dünnen Gelschichten höhere lonenkonzentrationen im Gel unterbringen lassen, ohne die Bevorzugung der Zahnoberfläche als Ort der heterogenen Nukleation zu gefährden. Um eine höhere Konzentration an Calcium- und Phosphat-Ionen in löslicher Form im Gel zu speichern, werden der Gelatine bevorzugt Aminosäuren zugefügt. Die Aminosäuren wirken durch ihre bindende Wechselwirkung mit den Mineralsalzen wie ein Depot und optimieren deren Verfügbarkeit. Es wird angenommen, dass bei lokaler Verarmung von Calcium und Phosphat infolge der Mineralisation die Aminosäuren durch Verschiebung des Gleichgewichtes Ionen freisetzen. Grundsätzlich sind alle Aminosäuren geeignet, Calcium- und Phosphat-Ionen zu binden, da jede als amphoteres Molekül sowohl über eine saure- als auch über eine basische Seitengruppe verfügt. Die optimale Wirksamkeit ist stark pH-abhängig. In direkter Umgebung der Mineralisationsfront sind infolge der säurefreisetzenden Apatitbildung besonders diejenigen Aminosäuren aktiv, die über saure Gruppen verfügen und Calcium freisetzen. Die Verfügbarkeit von Phosphat im Gel A kann bevorzugt durch die Zugabe von Aminosäuren mit zusätzlichen basischen Gruppen erhöht werden. Zur Erhöhung der Löslichkeit sind weiterhin alle Substanzen geeignet, die über Bindungsstellen für Calcium- und Phosphat-lonen verfügen, ohne diese auszufällen oder toxisch auf den menschlichen Organismus wirken. Darunter fallen beispielsweise Vitamine (z.B. Ascorbinsäure), Olygopeptide, Carbonsäuren und zwar insbesondere Fruchtsäuren wie Äpfelsäure, Zitronensäure oder Brenztraubensäure oder Komplexbildner wie EDTA. Diese Aufzählung ist einschließlich, aber nicht ausschließlich. Um den pH-Wert während der Mineralisation konstant zu halten, kann Essigsäure eingesetzt werden. Ebenso geeignet sind alle physiologisch verträglichen Puffersysteme, die im entsprechenden Pufferbereich zwischen 4 und 7 ihre maximale Wirksamkeit haben. Geeignet wären beispielsweise Dicarbonsäuren wie Bernsteinsäure, Malonsäure, oder Aminosäuren wie Glutaminsäure. Diese Aufzählung ist einschließlich, aber nicht ausschließlich.

DE1020040545847 beschreibt, dass eine Vorbenetzung der Zähne mit 0.05-1 N Natronlauge einen Einfluss auf Morphologie und Wachstumsgeschwindigkeit der Schichten hat. Es ist zu erwarten, dass bereits geringfügige Abweichungen in dieser Prozedur zu unterschiedlicher Benetzung mit Alkalie führt und das Resultat wesentlich beeinflusst.

Im Vorliegenden wird eine Methode beschrieben, bei der einer Alkalie geringe Mengen Gelatine zugefügt werden, bevorzugt 1-15 Gew.%, besonders bevorzugt 5-10 Gew.%, welche nach definierter Einwirkdauer mit definiertem Druck verblasen wird. Dadurch bilden sich reproduzierbar sehr dünne Filme auf der Zahnoberfläche, wodurch die initiale Mineralisation besonders begünstigt ist. Die Vorbehandlung kann erfindungsgemäß sowohl mit einer Flüssigkeit, einen zähflüssigen Gel oder einem erstarrten Gel, welches vor der Anwendung erwärmt wird, vorgenommen werden.

### Figur

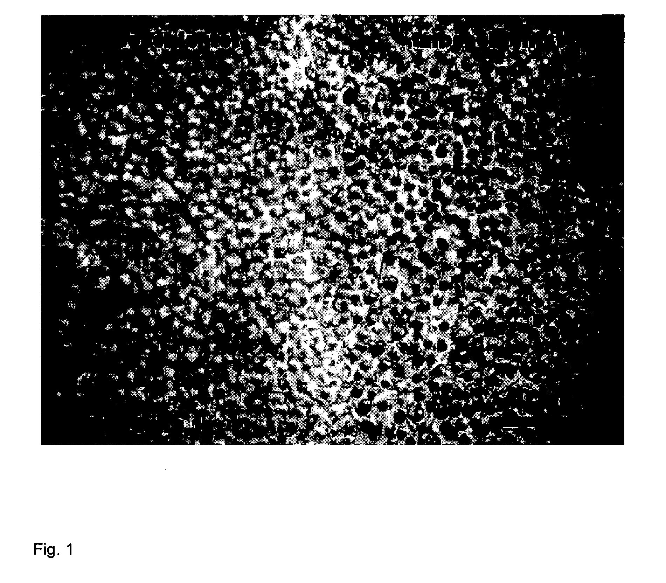

- Fig. 1: Lichtmikroskopische Aufnahme einer Zahnscheibe, deren linke Hälfte zwei Gelbehandlungszyklen unterworfen wurde; die rechte Hälfte war abgedeckt.

### Beispiel

Aus Molaren wird je eine 2 mm dicke Scheibe herausgesägt, die einen Dentin- und einen Schmelz-Bereich enthält. Alle Zahnproben werden poliert und je ein Molar für 10 Sekunden mit 25%iger Phosphorsäure behandelt, um die Dentintubuli freizulegen bzw. die native Schmelzstruktur sichtbar zu machen. Anschließend werden die Proben intensiv unter fließendem Wasser gewaschen. Die Zähne werden je 4x für 8 min. mit einem 35%igen Wasserstoffperoxid-Gel behandelt und im Anschluss mit Wasser gespült und einer Farbmessung unterzogen. Ein Zahn zeigt im Schmelzbereich eine mit der Oxidationsbehandlung in Zusammenhang gebrachte deutliche Rissbildung. Für die alkalische Vorbehandlung wird eine an Calciumhydroxid gesättigte, 1 N Natronlaugelösung mit 5 Gew.% Gelatine versetzt. Die Zahnscheiben werden mit dieser Lösung eingestrichen und die Feuchtigkeit wird im Anschluss gleichmäßig verblasen.

Für das phosphationenhaltige Gel wird eine Lösung hergestellt,die 0.6 mol/l Na₂HPO₄, 0.1 mol/I NaF, 0.3 mol/I Asparagin und 330 ml 2 N/I Essigsäure enthält. 16 ml der Lösung werden mit 6 g Glycerin und 10 g einer 300 Bloom Schweineschwarte-Gelatine unter Erwärmen zu einem dickflüsigen Gel verarbeitet. Mittels Rakel werden aus dem noch flüssigen Gel 300 µm starke Gelfilme ausgestrichen, getrocknet und anschließend in passgerechte Stücke geschnitten. Für das Calcium-Gel wird eine 1 molare Calciumchloridlösung angesetzt. Die Weiterverarbeitung entspricht der des Phosphatgels.
Die Zahnscheiben werden nun mit je einem Stück Phosphat-Gel und einem Stück Calciumgel bedeckt. Um die morphologische Änderung der Zahnoberfläche deutlich zu machen, wurde eine Scheibe zuvor zur Hälfte mit Parafilm abgedeckt, so dass sie nur hälftig remineralisieren kann. Die Proben werden im Klimaschrank bei 37°C und 95% Luftfeuchtigkeit aufbewahrt, täglich gewaschen und einer erneuten Gelbehandlung unterzogen. Abb. 1 zeigt eine lichtmikroskopische Aufnahme der halb bewachsenen Zahnscheibe nach dem 4. Tausch. Die Dentintubuli sind vollständig verschlossen bzw. die Polierspuren nicht mehr zu erkennen. Die Sprünge im Schmelz sind ebenfalls zugewachsen. Nach 5-fachem Geltausch werden die Proben gereinigt und für 40 Stunden in Kaffee eingelegt. Im Anschluss werden sie mittels Bürste unter fließendem Wasser gereinigt. Die gebleachte Probe zeigt eine deutlich dunklere Verfärbung als die bewachsene Probe. Um einen Vergleich zu ermöglichen, wird eine nicht behandelte Probe vor und nach der Einfärbung in Kaffee farbmetrisch vermessen. Um die Farbveränderung zu quantifizieren werden die Proben vor und nach der Einfärbung mit einem 2-Kanalspektralphotometer zur Farb- und Reflexionsmessung Spectraflash 600 Plus mit dem CIE L*a*b*-System mit der Blende 3mm X4SAV gegen weiß vermessen. Die Einfärbung führt bei allen Proben zu einer Verdunklung und Farbtonveränderung Richtung gelb und rot. Tabelle 1 zeigt die delta-Werte vor und nach der Einfärbung des Dentins für den gebleichten Zahn (Probe 1), den gebleichten und bewachsenen Zahn (Probe 2) und die ausschließlich eingefärbte Probe (Probe 3). Die Ergebnisse zeigen deutlich, dass sich die gebleachte Probe stärker verfärbt, als die unbehandelte Probe und deutlich stärker als die bewachsene Probe. Das heißt, die bewachsene Zahnscheibe zeigt sowohl gegenüber der gebleachten als auch der unbehandelten Probe eine schwächere Verfärbung.

**Tabelle 1**

| | ΔE | ΔL* | Δa* | Δb* | ΔC* | Δh |
|---|---|---|---|---|---|---|
| Probe 1 | 23.34 | -17.01 | 7.69 | 14.01 | 15.58 | -3.53 |
| Probe 2 | 5.71 | -2.53 | 4.84 | -1.66 | -0.78 | -5.06 |
| Probe 3 | 16.12 | -6.74 | 9.75 | 10.93 | 12.35 | -7.86 |

Die Figur 1 zeigt die Strukturveränderung durch die Gelbehandlung. Man erkennt sie als eine Nivellierung der Oberfläche.

## Patentansprüche

1. Mittel zum Schutz von Zahnflächen im Anschluss an konventionelle Bleichverfahren durch biomimetische Abscheidung von Fluorapatit, aufweisend
**A** mindestens einen vorkonfektionierten 50-1000 µm dicken Gelfilm A enthaltend
A1 mindestens einen Gelbildner,
A2 Wasser oder ein Gemisch aus Wasser und einem organischen Lösungsmittel,
A3 Phosphat- oder Hydrogenphosphationen,
A4 optional Fluorid,
A5 optional mindestens eine Aminosäure,
A6 ggf. eine Carbonsäure oder ein Puffersystem für einen der pH-Werte von 4 bis 7.
**B** mindestens eine 50 µm bis 5 mm dicken Gelfilm B enthaltend
B1 mindestens einen Gelbildner,
B2 Wasser,
B3 Calciumionen Ca²⁺.

2. Mittel gemäß Anspruch 1, bei dem
A3 0.01 - 2 mol pro Liter Gel eines Phosphats oder Hydrogenphosphats enthalten ist.

3. Mittel gemäß Anspruch 1, bei dem
B3 0.05 bis 3 mol pro Liter Gel Calciumionen Ca²⁺ enthalten sind.

4. Mittel nach Anspruch 1, wobei der Gelbildner Gelatine ist.

5. Mittel nach Anspruch 4, wobei die Gelatinekonzentration in A und B jeweils 1-15 Gew.% beträgt.

6. Mittel nach Anspruch 5, wobei die Gelatinekonzentration 5-10 Gew.% beträgt.

7. Mittel nach einem der vorstehenden Ansprüche, wobei das Phosphat oder Hydrogenphosphat als dissoziiertes Na₂HPO₄ (NH₄)₂HPO₄ oder K₂HPO₄ vorliegt.

8. Mittel nach einem der vorstehenden Ansprüche, wobei die Calciumionen als dissoziiertes CaCl₂ vorliegen.

## Geänderte Patentansprüche

### Geänderte Patentansprüche gemäss Regel 137(2) EPÜ.

**1.** Mittel zum Schutz von Zahnflächen im Anschluss an konventionelle Bleichverfahren durch biomimetische Abscheidung von Fluorapatit, aufweisend
**A** mindestens einen vorkonfektionierten 50-1000 µm dicken Gelfilm A enthaltend
A1 mindestens einen Gelbildner,
A2 Wasser oder ein Gemisch aus Wasser und einem organischen Lösungsmittel,
A3 Phosphat- oder Hydrogenphosphationen,
A4 Fluorid,
A5 optional mindestens eine Aminosäure,
A6 ggf. eine Carbonsäure oder ein Puffersystem für einen der pH-Werte von 4 bis 7.
**B** mindestens einen vorkonfektionierten 50 µm bis 5 mm dicken Gelfilm B enthaltend
B1 mindestens einen Gelbildner,
B2 Wasser,
B3 Calciumionen Ca²⁺.

**2.** Mittel gemäß Anspruch 1, bei dem
A3 0.01 - 2 mol pro Liter Gel eines Phosphats oder Hydrogenphosphats
enthalten ist.

**3.** Mittel gemäß Anspruch 1, bei dem
B3 0.05 bis 3 mol pro Liter Gel Calciumionen Ca²⁺
enthalten sind.

**4.** Mittel nach Anspruch 1, wobei der Gelbildner Gelatine ist.

**5.** Mittel nach Anspruch 4, wobei die Gelatinekonzentration in A und B jeweils 1-15 Gew.% beträgt.

**6.** Mittel nach Anspruch 5, wobei die Gelatinekonzentration 5-10 Gew.% beträgt.

**7.** Mittel nach einem der vorstehenden Ansprüche, wobei das Phosphat oder Hydrogenphosphat als dissoziiertes Na₂HPO₄ (NH₄)₂HPO₄ oder K₂HPO₄ vorliegt.

**8.** Mittel nach einem der vorstehenden Ansprüche, wobei die Calciumionen als dissoziiertes CaCl₂ vorliegen.
